# EUROPEAN PATENT SPECIFICATION

(11) **EP 0 751 226 B1**
(45) Date of publication and mention of the grant of the patent: **07.05.2008**
(21) Application number: 96108791.3
(22) Date of filing: 31.05.1996
(51) Int. Cl.: C12Q 1/68

(54) **Process for amplifying nucleic acid sequences**
Verfahren für die Amplifizierung von Nukleinsäure-Sequenzen
Procédé pour l'amplification de séquences d'acides nucléiques

(30) Priority: 28.06.1995 JP 16170195
(43) Date of publication of application: 02.01.1997
(73) Proprietor: Shimadzu Corporation, Kyoto-shi, Kyoto 604 (JP)
(72) Inventor: Nishimura, Naoyuki, c/o Shimadzu Corporation, Nakagyo-ku, Kyoto-shi, Kyoto 604 (JP); Nakayama, Tomoko, c/o Shimadzu Corporation, Nakagyo-ku, Kyoto-shi, Kyoto 604 (JP)
(74) Representative: Kilian, Helmut

(56) References cited:
- EP-A- 0 590 327
- BURCKHARDT J: "AMPLIFICATION OF DNA FROM WHOLE BLOOD" PCR METHODS & APPLICATIONS,US,COLD SPRING HARBOR LABORATORY PRESS, vol. 3, no. 4, 1 February 1994 (1994-02-01), pages 239-243, XP000456781 ISSN: 1054-9803
- PANACCIO M ET AL: "FOLT PCR: A SIMPLE PCR PROTOCOL FOR AMPLIFYING DNA DIRECTLY FROM WHOLE BLOOD" BIOTECHNIQUES,US,EATON PUBLISHING, NATICK, vol. 14, no. 2, 1 March 1993 (1993-03-01), pages 238-240,242,24, XP000345590 ISSN: 0736-6205
- DATABASE BIOSIS [Online] BIOSCIENCES INFORMATION SERVICE, PHILADELPHIA, PA, US; 1989 OLIVE D M ET AL: "POLYMERASE CHAIN REACTION ASSAY FOR DETECTION OF HUMAN CYTOMEGALOVIRUS" Database accession no. PREV198988039618 XP002146426 & JOURNAL OF CLINICAL MICROBIOLOGY, vol. 27, no. 6, 1989, pages 1238-1242, ISSN: 0095-1137

### SEQUENCE LISTING

(1) GENERAL INFORMATION:
   (iii) NUMBER OF SEQUENCES: 4
(2) INFORMATION FOR SEQ ID NO:1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid, synthetic DNA
   (iv) ANTI-SENSE: NO
   (xi) SEQUENCE DESCRIPTION: SEQ ID NO:1:
      GAAGAGCCAA GGACAGGTAC 20
(2) INFORMATION FOR SEQ ID NO:2:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid, synthetic DNA
   (iv) ANTI-SENSE: NO

   (xi) SEQUENCE DESCRIPTION: SEQ ID NO:2:
   GGAAAATAGA CCAATAGGCA G 21
(2) INFORMATION FOR SEQ ID NO:3:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid, synthetic DNA
   (iv) ANTI-SENSE: NO
   (xi) SEQUENCE DESCRIPTION: SEQ ID NO:3:
      CATGGGTACC AGCACACAAA GG 22
(2) INFORMATION FOR SEQ ID NO:4:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid, synthetic DNA
   (iv) ANTI-SENSE: NO
   (xi) SEQUENCE DESCRIPTION: SEQ ID NO:4:
      TCTACTTGTC CATGCATGGC TTC 23

## Description

### Background of the Invention

### Field of the Invention

This invention relates to a process for amplifying a specific nucleic acid sequence by PCR.

### Discussion of the Related Art

The PCR is an in vitro method for the enzymatic synthesis of specific DNA sequences, using two oligonucleotide primers that hybridize to opposite strands and flank the region of interest in the target DNA. By use of the PCR method, a target DNA can be amplified in an exponential fashion. The PCR is described in U.S. Patent Nos. 4,683,195 and 4,683,202 by K.B. Mullis et al.

Biotechniques 14, 238-243, 1993 Panaccio et al. furthermore discloses a method for direct PCR amplification from blood using FoLT PCR at a pH of 9.0. PCR methods and Applications 3, 239 - 243, 1994, J. Burckhardt furthermore discloses a method for direct PCR amplification from blood using PCR buffer and solution with buffer at pH 8.8.

The PCR is a highly sensitive test for the detection of DNA in specimens of various types, especially in those obtained from body fluids. Thus, PCR is a potentially powerful tool for use in the diagnosis of infectious diseases caused by microorganisms, hereditary diseases, cancers, etc. PCR is also suitable used for determining parent/child relationships and transplantation compatibility, where peripheral blood is frequently the specimen of choice.

One disadvantage to using PCR is that impurities such as pigments, proteins, sugars and unidentified contaminants inhibit the reaction. It is well known fact that a small amount of impurities derived from biological specimens strongly inhibits the activity of many DNA polymerases including *Taq* DNA polymerase produced by *Thermus aquaticus*, a representative thermostable DNA polymerase. In a conventional PCR procedure, a reaction mixture for PCR using *Taq* DNA polymerase is prepared as follows: First, a 10-fold concentrated PCR buffer is prepared using 1M Tris-HCl(pH 8.3 at 25°C), etc.; the concentrated buffer is freshly diluted 1:10 before use, to which deoxyribonucleotides (to make a concentration of 200µM each for dATP, dCTP, dGTP and dTTP), primers (to make a concentration of 0.2-1.0 µM), *Taq* DNA polymerase (to make a concentration of 2.5 units/100µl), and, according to necessity, other ingredients such as gelatin and surface active agents (NP 40, Tween, etc.) are added. To the reaction mixture thus prepared, a DNA fragment, or a specimen containing a DNA fragment is added as templates for PCR. In the conventional procedure, however, the presence of even a trace amount of impurities derived from biological specimens in the reaction mixture markedly inhibits PCR. Therefore, separation of the cells of animals and plants, bacteria, viruses, etc. (hereinafter referred to as "gene-containing materials") from materials to be tested and the subsequent extraction of DNA from the gene-containing materials are necessary prior to amplification by PCR. In order to overcome this inhibition, cellular lysis can be accomplished with enzymes, surface active agents or chaotropic agents and, traditionally, the subsequent extraction of the nucleic acid from the cellular lysate has been carried out using phenol or phenol-chloroform mixture. More recent methods of purifying the DNA include the removal of impurities by using ion exchange resins, glass filter, magnetic beads, or agents for protein flocculation.

One reagent kit for removing impurities is commercially marketed under the name of "COLLECTAGENE" (AMRAD Operations Pty Ltd., Victoria, Australia). Using this kit, the sample is pre-treated as follows:
1. Anticoagulant-treated whole blood of mammalian origin is mixed with Magnetic Particles in a tube, whereby the leukocyte cells and the Magnetic Particles are conjugated.
2. The cells conjugated with Magnetic Particles are washed with phosphate buffer.
3. The conjugated cells are lysed with proteinase, whereby the proteins are degraded and the DNA in the leukocyte cells is released.
4. The tube is then heated to inactivate the proteinase, resulting in a solution of leukocyte DNA which is ready for PCR.

Meanwhile, RNA-PCR is utilized for detecting and/or sequencing target RNA and thus has potential use for diagnostic tests and medical, industrial or environmental analyses. For example, RNA-PCR may be useful for detecting HCV (hepatitis C virus) which causes hepatitis, and HIV (human immunodeficiency virus) which causes AIDS.

In RNA-PCR, a complementary DNA (cDNA) is first synthesized using the target RNA as a template in the presence of reverse transcriptase. Then the cDNA is amplified by PCR. Under current technology, when treating the RNA obtained from living organisms, purification of the RNA is necessary. Basically, the RNA can be purified using the same purification method as for DNA.

In both DNA and RNA purifications, it is difficult to sufficiently remove the impurities as well as to recover DNA in a constant yield, and, thus, the succeeding DNA amplification frequently ends in failure, especially when the sample contains only small amount of DNA. In addition, the purification methods are so lengthy and complex that there is a high risk of contamination during the operation. Therefore, a simpler pretreatment which has less possibility of contamination has been in need.

The present invention addresses these and other problems associated with PCR amplification procedures and is believed to represent a significant advance in the art.

### Summary of the Invention

Accordingly, an object of the present invention is to provide a method for directly amplifying a gene of interest in a biological specimen, specifically to provide a method and reaction agents for PCR which allow direct addition of a biological specimen, in particular body fluids, to the PCR reaction system.

In summary, the object of this invention is to provide an improved process and reagents of PCR for amplifying genes, in particular DNAs contained in animal and plant tissues, body fluids, an excreta.

This object is obtained according to the present invention by the process claimed in claim 1.

### Brief Description of the Drawings

Fig. 1 shows a photograph of agarose gel electrophoresis carried out for detecting DNA amplified in Experimental Example 2.
Fig. 2 shows a photograph of agarose gel electrophoresis carried out for detecting DNA amplified in Experimental Example 3.
Fig. 3 shows a photograph of agarose gel electrophoresis carried out for detecting DNA amplified in Experimental Example 4.

### Detail Description of the Invention

The term "biological specimens" as used in the present application refers to specimens obtained from animals and plants, which include tissues of animals and plants, body fluids, and excreta. The body fluids include, but are not limited to, blood and saliva.

The term "gene-containing materials" used herein includes cells of animals and plants, bacteria, and viruses. The cells of animals include, but are not limited to, leukocytes isolated from blood.

The PCR reaction mixture used in the present invention usually contains a pH buffer, salts (MgCl₂ and KCl, etc.), primers, deoxyribonucleotides, and a DNA polymerase. Other salts than MgCl₂ and KCl may be used according to necessity. Other ingredients, for example, surface active agents such as Nonidet P-40 and polyoxyethylene sorbitans; proteins such as gelatin and albumin; and dimethylsulfoxide may be added to the reaction mixture in some cases.

A preferred buffer for use in the present invention consists of Tris (hydroxymethyl) aminomethane and a mineral acid, such as hydrochloric acid, nitric acid, and sulfuric acid. Among these acids, a preference is given to hydrochloric acid. Other various buffers may also be used. For example, CAPSO (3-N-cyclohexylamino-2-hydroxypropanesulfonic acid) or CHES (2-(cyclohexylamino)ethanesulfonic acid), in combination with sodium hydroxide or potassium hydroxide, can be used. The buffer solution with an adjusted pH is used in a concentration of from 10mM to 100mM in the reaction mixture for PCR.

The term "primer" as used in the present application refers to an oligonucleotide which is capable of acting as a point of initiation of synthesis in the presence of DNA, deoxyribonucleotides, a DNA polymerase, etc. The primers are preferably single stranded but may be double stranded. If the primers are double stranded, the strands are separated prior to amplification reaction.

The oligonucleotide primers can be prepared by methods which are well known in the art or can be obtained from a biological source.

The term "nucleic acid" as used in the present application refers to double or single stranded DNA or RNA or a double stranded DNA-RNA hybrid. The nucleic acids may be obtained from any source. Any nucleic acid can be amplified using the present invention as long as a sufficient number of bases at both ends of the sequence are known so that oligonucleotide primers, which will hybridize to the strands of the nucleic acid to be amplified, can be prepared.

The term "polymerase" as used in the present application refers to any enzymes which can synthesize a DNA by primer extension reaction. Suitable polymerases include, but are not limited to, *E*. *coli* DNA polymerase I, Klenow fragment of *E*. *coli* DNA polymerase I, T4 DNA polymerase, *Taq* DNA polymerase, *T*. *litoralis* DNA polymerase, *Tth* DNA polymerase, *Pfu* DNA polymerase, and reverse transcriptase.

The term "thermostable" as used in the present application refers to a property of substance which retains activity at an elevated temperature, preferably 65°C-95°C.

In the PCR reaction systems where a blood specimen is directly added to the PCR reaction mixture (PCR with whole blood), which are suitable for analyses of genomic DNA in HLA typing and diagnoses of hereditary diseases, the initial pH of the reaction mixture is not less than 8.5, preferably in the range of from 8.5 to 9.0. When the initial pH of the PCR reaction mixture is adjusted to an optimal level around pH 8.8, the blood added to the reaction mixture does not inhibit the PCR up to the whole blood content of 2.5% in the reaction mixture.

In the PCR reaction system where isolated leukocytes are directly added to the PCR reaction mixture (PCR with leukocytes), which are suitable for the detection of a small amount of DNA in peripheral leukocytes, e.g., detection of HIV provirus DNA, the initial pH of the reaction mixture is not less than 8.5, preferably in the range of from 8.5 to 9.2. When the initial pH of the reaction mixture is adjusted in this range, 100 or less molecules of HIV DNA mixed with 100000 molecules of leukocyte DNA can be detected. In particular, when the initial pH is adjusted to an optimal level around pH 9.0, only 10 molecules of HIV DNA can successfully be detected.

When a specimen of saliva is directly added to the reaction mixture for PCR (PCR with saliva), the initial pH of the reaction mixture is adjusted to not less than 8.5, preferably not less than 8.8. In the above pH range, cellular DNA in the saliva specimen can stably be amplified. In particular, when the initial pH of the PCR reaction mixture is increased to 9.4, PCR is successfully carried out in the reaction mixture which contains as high as 8% saliva.

The reaction mixture for PCR used in the present invention may be prepared by an experimenter before use. Also, the present invention can more readily and safely be carried out by using a kit which consists of all or part of the component reagents of the reaction mixture.

The reagent kit is characterized in that it is used to amplify a target gene in a specimen by PCR, and comprises a pH buffer, primers, deoxyribonucleotides, and a thermostable polymerase, and that the pH buffer is capable of maintaining the initial pH of the reaction mixture for PCR within the range of from 8.5 to 9.5 at 25°C.

According to the present invention, specific DNA amplification can be successfully carried out by directly adding a biological specimen, in particular specimens obtained from body fluids, to the reaction system without pretreatment or after only rough treatment for isolation. That is, in the present invention, a specimen from body fluids, such as whole blood, plasma, serum and saliva, can be mixed with the reaction mixture for PCR without any pretreatments. During the thermal cycle of PCR, any of the above specimens mixed with the reaction mixture for PCR is placed under high temperature for denaturation, and the cells in the specimen is lysed so that the DNA in the cells is released and can access the primers and other necessary reagents for PCR in the reaction mixture. Though components of saliva or blood are present in the reaction mixture owing to lack of sufficient pretreatment of the specimen, such impurities do not hamper the DNA amplification in the improved method of the present invention.

Thus, according to the present invention, pretreatment of the specimens for PCR can be simplified, thereby facilitating the process for amplifying and detecting a specific gene by PCR without risks of contamination. Furthermore, by use of the reagent kit of the present invention, the above-mentioned improved process for PCR of the present invention is more readily and safely carried out.

The following Experimental Examples illustrate the present invention but are not intended to limit the invention in any manner.

### EXAMPLES

### Experimental Example 1: Measurement of pH of PCR reaction mixture

The pH of the reaction mixture actually used for PCR was measured. That is, at first, 1M Tris-HCl of varying pH values were prepared. And then 100 mM Tris-HCl buffers containing 10-fold dilution of each 1M Tris-HCl, 500mM KCl and 15mM MgCl₂ (hereinafter simply referred to as "concentrated PCR buffer") were prepared. Then, the reaction mixtures for PCR, each of which was a mixture of 10mM Tris-HCl, 50mM KC1, 1.5mM MgCl₂, 200µM each of dATP, dCTP, dGTP, and dTTP, 1.0µM each of primers, and 2.5 units/100µl of Taq DNA polymerase (Takara Taq: Takara shuzo), were prepared using the concentrated PCR buffers. The pH values of the 1M Tris-HCl, concentrated PCR buffers, and reaction mixtures for PCR are listed in Table 1. The primers used were two oligonucleotides having the following sequences:
P1: 5' GAAGAGCCAAGGACAGGTAC 3' (SEQ ID NO:1)
P2: 5' GGAAAATAGACCAATAGGCAG 3' (SEQ ID NO:2)
The P1 primer is homologous to the plus-strand and the P2 primer is homologous to the minus strand of human beta-globin coding region. With these primers, an amplified product of 408 bp sequence contained within the human beta-globin gene is to be obtained. (Saiki, R.K., Gelfand, D.H., Stoffel, S., Scharf, S.J., Higuchi, R., Horn, G.T., Mullis, K.B., and Erlich, H.A. (1988), Science, 239, 487-491).

From Table 1, it is clear that the pHs of the PCR reaction mixtures become lower than the pHs of the 1M Tris-HCl or the pHs of the concentrated PCR buffers. For example, a 1M Tris-HCl of pH 8.3, the pH most commonly used for PCR, afforded a concentrated PCR of the same pH (pH 8.3), but the pH of the PCR reaction mixture prepared from the concentrated PCR buffer of pH 8.3 was lowered to 8.1. Similarly, the 1M Tris-HCl having a pH of from 8.5 to 10.0 afforded the concentrated PCR buffers of almost the same pH, whereas the pH of the reaction mixtures for PCR were lower than the starting 1M Tris-HCl by 0.2 to 0.8. Also, the pH of 1M Tris solution without addition of HCl was 11, and decreased to 10.2 in the concentrated PCR buffer and further to 9.4 for the reaction mixture for PCR prepared using the concentrated PCR buffer.

**Table 1**

| Varying pH of Tris-HCl, 10-fold concentrated PCR buffers, and reaction mixtures for PCR | | |
|---|---|---|
| 1M Tris-HCl | Concentrated PCR buffers | Reaction mixtures for PCR |
| pH 8.3 | pH 8.3 | pH 8.1 |
| pH 8.5 | pH 8.6 | pH 8.3 |
| pH 8.8 | pH 8.9 | pH 8.5 |
| pH 9.1 | pH 9.1 | pH 8.8 |
| pH 9.5 | pH 9.5 | pH 9.0 |
| pH 10.0 | pH 9.9 | pH 9.2 |
| pH 11.0 | pH 10.2 | pH 9.4 |

Next, the relationship between pH and the degree of DNA amplification was studied. In these Examples, several reaction mixtures of varying pH in the range of from 8.1 to 9.4 were prepared by changing the pH with an increment of 0.2 to 0.3, to which a blood or saliva specimen was directly added to amplify a target gene of the gene-containing materials in the specimen.

### Experimental Example 2: PCR with Human Whole Blood Specimen

To the reaction mixtures for PCR of varying pH, 10 to 0.63 µl of human whole blood was added to make a total volume of 100 µl, and PCR was carried out. The reaction mixtures for PCR prepared in Experimental Example 1 were used.

After preincubation at 94°C for 2 minutes, 40 cycles of PCR using the following temperature profile were performed:

| | |
|---|---|
| Denaturation | 94°C, 1 minute |
| Primer Annealing | 55°C, 1 minute |
| Primer Elongation | 72°C, 1 minute |

The PCR cycles were terminated with a final elongation at 72°C for 7 minutes.

Agarose gel electrophoresis was employed to detect the amplified DNA. After the PCR, 5 µl of each reaction mixture was applied to a 3% agarose gel and separated by electrophoresis in TAE buffer (40mM Tris-acetate, 1mM EDTA, pH 8.0) containing 0.5 µg/ml of ethidium bromide.

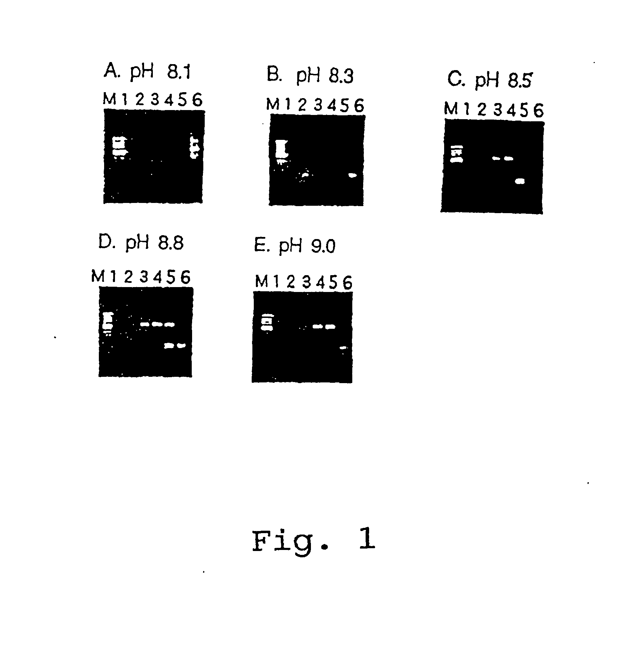

The results of Experimental Example 2 are shown in Fig. 1. In Fig. 1, the letter M represents a molecular weight marker which is Hinc II-digested φx174 RF DNA, and the numbers 1 to 6 represent different amounts of the human whole blood added to the reaction mixture for PCR, namely:

| Number | Amounts of human whole blood |
|---|---|
| 1 | 10 µl |
| 2 | 5 |
| 3 | 2.5 |
| 4 | 1.25 |
| 5 | 0.63 |
| 6 | 0 |

When PCR was carried out with a standard PCR reaction mixture (pH 8.1) prepared with 1M Tris-HCl of pH 8.3, there was no detectable band specific for the beta-globin gene in any lanes (Fig.1 A). Similarly, when the PCR was carried out with a reaction mixture at pH 8.3, there was no detectable band in any lane (Fig.1 B).

When the pH of the reaction mixture for PCR was increased to 8.5, detectable bands specific for the beta-globin gene were obtained in lanes 3 (whole blood amount of 2.5 µl) and 4 (1.25 µl) (Fig. 1 C). When the reaction mixtures of pH 8.8 and pH 9.0 were used, detectable bands specific for the target gene were obtained in lanes 3, 4 and 5 (2.5, 1.25 and 0.63 µl; Fig. 1 D) and lanes 4 and 5 (1.25 and 0.63 µl; Fig. 1E), respectively.

The above results indicate that when PCR is carried out with buffers at a higher pH than the pH commonly used, the amplification of a target gene can take place by directly mixing a blood specimen with a reaction mixture for PCR. It is also indicated that the optimal pH of the reaction mixture for PCR with a whole blood specimen is around 8.8.

### Experimental Example 3: PCR with Leukocytes Collected after Hemolysis

After 23.7 µl of human blood containing 1.4 x 10⁵ leukocytes was subjected to hemolytic treatment, the lysate was centrifuged. The supernatant was removed to collect residual leukocyte pellet. To the leukocyte pellet collected, HIV DNA of varying copies (1000, 100, 10, and 0 copies) and reaction mixtures for PCR of varying pH were added to make a total volume of 100 µl, and PCR was carried out. The procedures of PCR and the detection methods as described in Experimental Example 2 were used, and the reactions mixtures for PCR were the same as those used in Experimental Example 1 except that the primers were replaced with the following oligonucleotides:

### P3: 5' CATGGGTACCAGCACACAAAGG 3' (SEQ ID NO:3)

### P4: 5' TCTACTTGTCCATGCATGGCTTC 3'(SEQ ID NO:4)

The P3 primer is homologous to the plus-strand and the P4 primer is homologous to the minus strand of the HIV pol region. With these primers, an amplified product of 244 bp is to be obtained.

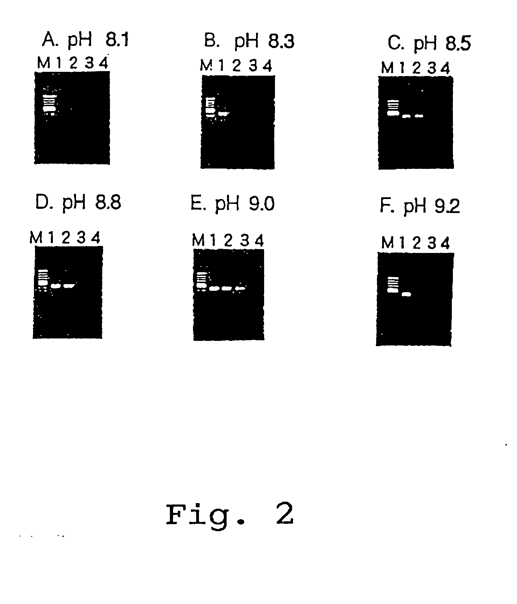

The results of Experimental Example 3 are shown in Fig. 2. In Fig. 2, the letter M represents a molecular weight marker which is Hinc II-digested φx174 RF DNA, and the numbers 1 to 4 represent different copies of HIV DNA added to the reaction mixture for PCR, namely:

| Number | Copies of HIV DNA |
|---|---|
| 1 | 1000 copies |
| 2 | 100 |
| 3 | 10 |
| 4 | 0 |

When PCR was carried out with a standard PCR reaction mixture (pH 8.1) prepared with 1M Tris-HCl of pH 8.3, there was no detectable band specific for the HIV DNA in any lanes (Fig.2 A). Similarly, when the PCR was carried out with a reaction mixture of pH 8.3, amplified DNA specific for the HIV DNA was detected only in lane 1 (1000 copies of HIV DNA, Fig.2 B). With increasing the pH of the reaction mixture up to pH 9, the sensitivity of PCR reaction was increased. That is, the detectable number of DNA copies was 100 copies at pH 8.5 (Fig. 2 C), which was decreased to 10 copies at pHs 8.8 and 9.0 (Figs. D and E).

The amounts of the PCR amplified product were compared between pH 8.8 and pH 9.0, by comparing the intensity of the bands appearing on the agarose gel. As a result, the amount of the amplified product obtained at pH 9.0 was greater than that obtained at pH 8.8. When the pH of the reaction mixture was increased to pH 9.2, the intensity of the band obtained with 100 copies of the DNA was markedly weak, though a highly intense band was obtained with 1000 copies.

The above results indicate that PCR carried out with buffers adjusted at a higher pH than the pH commonly used makes it possible to amplify a target gene, even when collected peripheral leukocytes are directly mixed with a reaction mixture for PCR. It is also indicated that the optimal pH of the reaction mixture for PCR with peripheral blood leukocytes is around 9.0.

### Experimental Example 4: PCR with Human Saliva

To PCR reaction mixtures of varying pH, 4 to 0.5 µl of human saliva was added to make a total volume of 50 µl, and PCR was carried out. The procedures of PCR and the detection methods as described in Experimental Example 2 were used, and the reaction mixtures for PCR were the same as those used in Experimental Example 1.

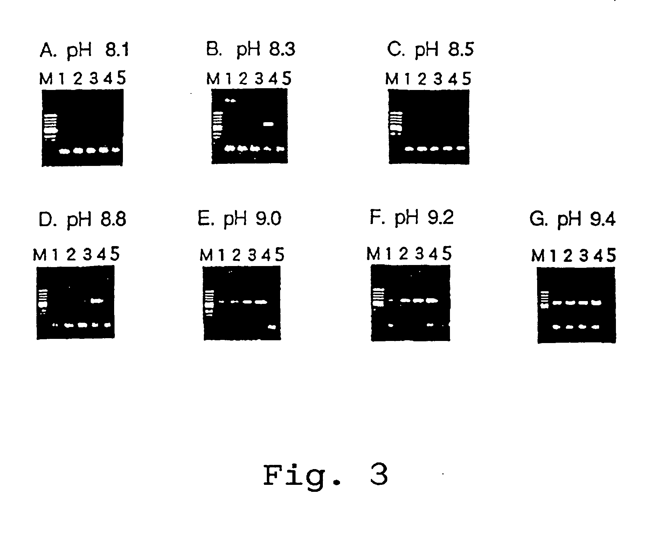

The results of Experimental Example 4 are shown in Fig. 3. In Fig. 3, the letter M represents a molecular weight marker which is Hinc II-digested φx174 RF DNA, and the numbers 1 to 5 represent different amounts of human saliva added to the reaction mixture for PCR, namely:

| Number | Amounts of Saliva |
|---|---|
| 1 | 4 µl |
| 2 | 2 |
| 3 | 1 |
| 4 | 0.5 |
| 5 | 0 |

When PCR was carried out with a standard PCR reaction mixture (pH 8.1) prepared with 1M Tris-HCl of pH 8.3, there was no detectable band specific for the beta-globin gene in any lanes (Fig.3 A). When the PCR was carried out with a reaction mixture of pH 8.3, amplified DNA specific for the beta-globin gene was detected only in lane 4 (saliva amount of 0.5 µl, Fig.3 B), but the band of amplified DNA became again undetectable in any lanes when a reaction mixture of pH 8.5 was used (Fig.3 C).

However, when PCR was carried out with a PCR reaction mixture of pH 8.8 or higher pH, amplified DNA was detected in more lanes. Specifically, the reaction mixtures of pH 8.8 and 9.0 were used, detectable amounts of a PCR amplified product could be obtained with 0.5 µl or higher amounts of saliva specimens (Figs. 3D and 3E). With further increase in pH of the reaction mixture for PCR, the amount of the amplified product produced became higher. When a reaction mixture of pH 9.4 prepared with 1M Tris solution without containing HCl was used, the amplified product could be detected in all the lanes in sufficiently detectable amounts by electrophoresis (Fig. 3G).

The above results indicate that when PCR carried out at a higher pH than the pH commonly used makes it possible to amplify a target gene, even when a specimen of human saliva is directly mixed with a reaction mixture for PCR. It is also indicated that the optimal pH of the reaction mixture for PCR with a specimen of human saliva is not less than 9.4.

## Claims

1. A process for amplifying a specific nucleic acid sequence by PCR wherein a gene-containing material collected from a biological specimen or a biological specimen is directly applied to a PCR reaction system and wherein an initial pH at 25°C of a reaction mixture for PCR consisting of a pH buffer, salts, primers, deoxyribonucleotides and a DNA polymerase, optionally surface active agents, proteins and dimethylsulfoxide is adjusted in the range of 8.8 to 9.5.

2. A process according to claim 1, wherein the initial pH at 25°C of the reaction mixture is adjusted in the range of 9.0 to 9.5.

3. The process according to claim 1 or 2, wherein the biological specimen is obtained from a body fluid and wherein PCR is carried out using *Taq* DNA polymerase.

4. The process according to claim 3, wherein the body fluid is blood or saliva.

## Patentansprüche

1. Verfahren zur Amplifikation einer spezifischen Nukleinsäuresequenz durch PCR, wobei ein Gen-enthaltendes Material, welches von einer biologischen Probe gewonnen wurde, oder eine biologische Probe direkt einem PCR-Reaktionssystem unterzogen wird, und wobei der anfängliche pH-Wert bei 25 °C der PCR-Reaktionsmischung, enthaltend einen pH-Puffer, Salze, Primer, Desoxyribonukleotide und eine DNA-Polymerase, optional grenzflächenaktive Agenzien, Proteine und Dimethylsulfoxid, in dem Bereich von 8,8 bis 9,5 eingestellt wird.

2. Verfahren gemäß Anspruch 1, wobei der anfängliche pH-Wert bei 25°C der Reaktionsmischung in dem Bereich von 9,0 bis 9,5 eingestellt wird.

3. Verfahren gemäß Anspruch 1 oder 2, wobei die biologische Probe aus einer Körperflüssigkeit gewonnen wird, und wobei die PCR unter Verwendung von Taq-DNA-Polymerase durchgeführt wird.

4. Verfahren gemäß Anspruch 3, wobei die Körperflüssigkeit Blut oder Speichel ist.

## Revendications

1. Procédé pour l'amplification d'une séquence spécifique d'acides nucléiques par PCR dans lequel un matériau contenant un gène collecté à partir d'un spécimen biologique ou un spécimen biologique est appliqué directement à un système de réaction de PCR et dans lequel un pH initial à 25°C d'un mélange réactionnel pour PCR consistant en un tampon de pH, des sels, des amorces, des désoxyribonucléotides et une ADN polymérase, éventuellement des agents tensioactifs, des protéines et du diméthylsulfoxyde, est ajusté dans la plage de 8,8 à 9,5.

2. Procédé selon la revendication 1, dans lequel le pH initial à 25°C du mélange réactionnel est ajusté dans la plage de 9,0 à 9,5.

3. Procédé selon la revendication 1 ou 2, dans lequel le spécimen biologique est obtenu à partir d'un fluide corporel et dans lequel la réaction de PCR est effectuée en utilisant une ADN Taq polymérase.

4. Procédé selon la revendication 3, dans lequel le fluide corporel est le sang ou la salive.
